# EUROPÄISCHE PATENTSCHRIFT

(11) **EP 4 753 783 B1**
(45) Veröffentlichungstag und Bekanntmachung des Hinweises auf die Patenterteilung: **04.11.2026**
(21) Anmeldenummer: 24768519.1
(22) Anmeldetag: 06.09.2024
(51) Int. Cl.: A61M 16/04, A61M 16/08

(54) **KONNEKTORMITTEL, PFLASTER, TRACHEALKANÜLE UND VERWENDUNG EINES KONNEKTORMITTELS**
CONNECTOR MEANS, TAPE, TRACHEAL CANNULA, AND USE OF A CONNECTOR MEANS
MOYEN CONNECTEUR, RUBAN, CANULE TRACHÉALE ET UTILISATION D'UN MOYEN CONNECTEUR

(30) Priorität: 07.09.2023 DE 102023124092
(43) Veröffentlichungstag der Anmeldung: 10.06.2026
(73) Patentinhaber: Andreas Fahl Medizintechnik-Vertrieb GmbH, 51149 Köln (DE)
(72) Erfinder: FAHL, Andreas, 51149 Köln (DE)
(74) Vertreter: Geskes, Christoph
(86) Internationale Anmeldenummer: PCT/EP2024/074921
(87) Internationale Veröffentlichungsnummer: WO 2025/051911

(56) Entgegenhaltungen:
- EP-A2- 3 153 201
- WO-A1-2005/023360
- DE-U1- 202005 020 032
- DE-U1- 202008 002 602

## Beschreibung

Die Erfindung betrifft ein Konnektormittel, ein Pflaster, eine Trachealkanüle und eine Verwendung eines Konnektormittels.

Aus dem Stand der Technik sind Konnektormittel für Tracheostomahilfsmittel allgemein bekannt. EP 2 953 674 B1 offenbart ein Tracheostomapflaster mit einer Aufnahme für ein Tracheostomahilfsmittel wie einen Feuchtetauscher oder ein Sprechventil, wobei das Tracheostomahilfsmittel mittels einer Klemmverbindung in der Aufnahme befestigbar ist.

Aus dem Stand der Technik sind des weiteren folgende Dokumente bekannt, nämlich DE 20 2005 020032 U1, DE 20 2008 002602 U1 sowie WO 2005/023360 A1.

Aufgabe der vorliegenden Erfindung ist es, ein verbessertes Konnektormittel zur Verfügung zu stellen. Insbesondere ist Aufgabe der Erfindung, ein Konnektormittel zur Verfügung zu stellen, das ein Tracheostomahilfsmittel sicher hält. Weiter ist es Aufgabe der Erfindung, ein Konnektormittel zur Verfügung zu stellen, mittels dem eine große Anzahl von unterschiedlichen Tracheostomahilfsmitteln aufnehmbar sind.

Die Aufgabe wird erfindungsgemäß gelöst mittels einem Konnektormittel zur Verbindung mit einem Tracheostomahilfsmittel, wobei das Konnektormittel einen Konnektorring umfasst, wobei der Konnektorring einen ersten Schnappring und zumindest einen zweiten Schnappring umfasst, wobei der erste Schnappring und der zweite Schnappring auf einer Innenoberfläche des Konnektorrings ausgebildet sind und wobei der erste Schnappring einen ersten Innendurchmesser und der zweite Schnappring einen zweiten Innendurchmesser aufweist, wobei der erste Innendurchmesser größer ist als der zweite Innendurchmesser, wobei der erste Schnappring und der zweite Schnappring eine wulstförmige Materialanhäufung umfassen und elastisch verformbar sind und wobei der erste Schnappring distal, das heißt bei üblichen Gebrauch des Konnektormittels in einer Richtung vom Körper einer Person weg gerichtet, von dem zweiten Schnappring angeordnet ist.

Weiterhin wird die Aufgabe erfindungsgemäß gelöst mittels einem Pflaster mit einem oben genannten Konnektormittel, wobei ein Anschlussteil auf einer distalen Seite des Pflasters angeordnet ist.

Weiterhin wird die Aufgabe erfindungsgemäß gelöst mittels einer Trachealkanüle mit einem oben genannten Konnektormittel, wobei ein Konnektorring an einem distalen Ende eines Kanülenrohrs angeordnet ist.

Weiterhin wird die Aufgabe erfindungsgemäß gelöst mittels einer Verwendung eines wie oben genannten Konnektormittels zur Verbindung mit Tracheostomahilfsmitteln mit unterschiedlichen Außendurchmessern.

Es wird ein Konnektormittel zum Anschluss eines Tracheostomahilfsmittels vorgeschlagen, wobei das Konnektormittel einen Konnektorring umfasst. Der Konnektorring umfasst einen ersten Schnappring und zumindest einen zweiten Schnappring. Der erste Schnappring und der zweite Schnappring sind auf einer Innenoberfläche des Konnektorrings ausgebildet. Der erste Schnappring und der zweite Schnappring weisen unterschiedliche Innendurchmesser auf.

Bei operativen Eingriffen im oberen Atemtrakt kann das Anlegen einer künstlichen Atem-Öffnung, dem Tracheostoma, in der Luftröhre notwendig sein, damit unter Umgehung von Mundraum und Kehlkopf Luft direkt in die Lunge eingeatmet werden kann. Bei laryngektomierten, also kehlkopflosen Personen, bei welchen der Kehlkopf operativ entfernt wurde, muss das Tracheostoma dauerhaft offen gehalten und stabilisiert werden, wozu insbesondere Trachealkanülen, in der Regel aus einer Außen- und einer Innenkanüle bestehend, in das Tracheostoma eingesetzt werden. Aber auch der Einsatz so genannter "Tracheostoma-Button" ist möglich - insbesondere für Patienten, die ansonsten keine Trachealkanüle mehr benötigen.

Des Weiteren können in das Tracheostoma auch so genannte "Shunt-Ventile" eingesetzt werden, welche eine Wiederherstellung der Stimme ermöglichen. Schließlich können in das Tracheostoma auch Filtersysteme eingesetzt werden, sowohl bei Tracheotomierten als auch bei Laryngektomierten. Derartige Filtersysteme bestehen entweder aus einem Pflaster mit einem eingesetzten Filter oder aus einem üblicherweise selbstklebenden Anschlussteil beziehungsweise einer Basisplatte - in aller Regel aus Kunststoff gebildet -, in welche Filter unterschiedlichster Art einsetzbar sind.

Zu den Filtersystemen, welche für laryngo-tracheale Hilfsmittel wie Trachealkanülen, Shunt-Ventile, Tracheostoma-Button und Filtersysteme mit Pflaster oder Basisplatte eingesetzt werden, zählen die so genannten "Feuchte- und Wärmeaustauscher", auch "künstliche Nase" genannt. Diese dienen dazu, die bei laryngektomierten und auch tracheotomierten Patienten fehlenden Regulationsmechanismen zur Erwärmung und Befeuchtung der Atemluft nachzubilden und ein In-Kontakt-Bringen der Luftröhre mit verstärkt trockener, kalter und nicht gefilterter Luft zu vermeiden. Denn durch die hierdurch hervorgerufene Reizung tritt eine erhöhte Schleimproduktion mit der nachfolgenden Gefahr der Verborkung auf. Durch Feuchte- und Wärmeaustauscher wird nun die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird.

Feuchte- und Wärmeaustauscher für Laryngektomierte und Tracheotomierte weisen einen Filterkörper auf - in aller Regel aus Papier oder Schaumstoff gebildet - durch welchen die ein- und ausgeatmete Luft geleitet wird. Beim Ausatmen hält der Filterkörper Feuchtigkeit zurück, welche dann beim Einatmen in die Luftröhre transportiert wird. Aus dem Stand der Technik bekannte künstliche Nasen, auch "HME" (humid and moisture exchanger) genannt, gibt es in einer großen Vielzahl unterschiedlicher Ausbildungen für unterschiedliche Adapter. Der Universaladapter gemäß DIN EN ISO 5356-1:2015-06 weist dabei einen Durchmesser von 15 mm und 22 mm auf, es sind jedoch aus dem Stand der Technik auch weitere Adapter mit insbesondere größeren Durchmessern, beispielsweise 16 mm und 23 mm, bekannt. Der Universaladapter von beispielsweise 15 mm oder 16 mm wird insbesondere bei Trachealkanuelen eingesetzt, wohingegen Tracheostoma-Button und Filtersysteme - insbesondere mit einer selbstklebenden Basisplatte oder mit einem Pflaster ausgebildet - einen größeren Adapter-Durchmesser, wie beispielsweise 22 mm oder 23 mm, aufweisen.

Ein Tracheostomahilfsmittel kann beispielsweise ein Wärme-Feuchtigkeitsaustauscher oder ein Sprechventil oder eine Kombination beider sein. In einer Ausgestaltung ist vorgesehen, dass das Tracheostomahilfsmittel, dass mittels des Konnektormittels aufnehmbar ist, zumindest abschnittsweise einen Außendurchmesser von etwa 11 mm bis etwa 25 mm, bevorzugt etwa 21 mm bis etwa 24 mm, weiter bevorzugt etwa 11,5 mm, weiter bevorzugt etwa 15 mm, weiter bevorzugt etwa 16 mm, weiter bevorzugt etwa 22 mm, weiter bevorzugt etwa 23 mm, aufweist.

Beispielhafte Aufzählungen wie die vorstehende sind im Sinne der Offenbarung als nicht abschließend anzusehen, sondern können im Rahmen des allgemeinen Fachwissens ergänzt werden.

Vorteilhaft an dem vorgeschlagenen Konnektormittel ist, dass dieses bevorzugt mittels der zumindest zwei Schnappringe Tracheostomahilfsmittel unterschiedlicher Größe aufnehmen kann. Insbesondere kann das Konnektormittel zumindest ein genormtes Tracheostomahilfsmittel und zumindest ein weiteres, nicht genormtes Tracheostomahilfsmittel aufnehmen. Das Konnektormittel umfasst bevorzugt ein proximales Anschlussteil. In einer Ausgestaltung ist vorgesehen, dass das Konnektormittel ein Anschlussteil umfasst, das als eine Basisplatte ausgebildet ist. In einer bevorzugten Ausführungsform ist das Anschlussmittel beziehungsweise die Basisplatte eine am proximalen Ende des Konnektorrings an dessen Außenwandung angeordnete Ring-Scheibe. Bevorzugt erstreckt sich das Anschlussmittel am proximalen Ende des Konnektorrings radial nach außen. Bevorzugt ist proximal an dem Anschlussmittel beziehungsweise der Basisplatte ein Klebemittel, beispielsweise eine Klebefolie bevorzugt mit einer abziehbaren Schutzfolie, angeordnet. Konnektormittel mit Klebemittel bilden dann beispielsweise eine Tracheostomaplaster.

In einer weiteren Ausgestaltung ist vorgesehen, dass proximal des Konnektorrings eine Kanüle angeordet ist. Beispielsweise ist die Kanüle ein Kanülenrohr. Beispielsweise bilden Konnektormittel und Kanülenrohr einen Tracheostomabutton oder eine Trachealkanüle.

Werden im Rahmen der Beschreibung der Offenbarung Richtungsangaben verwendet, so sind diese in Bezug auf den üblichen Gebrauch des Konnektormittels zu verstehen. Richtungsbezeichnungen werden wie in der Anatomie üblich verwendet. Unter dem Begriff "distal" wird im Sinne der vorliegenden Offenbarung in Bezug auf ein Merkmal der erfindungsgemäßen Vorrichtung eine Anordnung bzw. Verwendung desselben fern oder auch abgewandt oder gegenüberliegend eines Tracheostomapflasters oder einer Trachealkanüle, allgemeiner einer Hautoberfläche einer Person, die insbesondere solche Befestigungsmittel für die offenbarungsgemäß Vorrichtung trägt, verstanden. Unter distal ist vorzugsweise eine Richtung vom Körper der Person weg zu verstehen. Unter dem Begriff "proximal" wird im Sinne der vorliegenden Offenbarung in Bezug auf ein Merkmal der offenbarungsgemäßen Vorrichtung eine Anordnung oder Verwendung desselben nah oder auch zugewandt oder benachbart eines Tracheostomapflasters oder einer Trachealkanüle, allgemeiner einer Hautoberfläche einer Person, die insbesondere solche Befestigungsmittel für die offenbarungsgemäße Vorrichtung trägt, verstanden. Unter proximal ist vorzugsweise eine Richtung zum Körper der Person hin zu verstehen.

Das Konnektormittel umfasst einen Konnektorring. Bevorzugt ist der Konnektorring zylindrisch ausgestaltet. Bevorzugt umfasst der Konnektorring einen Außenmantel. Weiter bevorzugt umfasst der Konnektorring eine Innenoberfläche. Bevorzugt erstreckt sich die Innenoberfläche von einem proximalen Ende des Konnektorrings zu einem distalen Ende des Konnektorrings auf dessen Innenseite. In einer Ausgestaltung ist vorgesehen, dass das Anschlussteil auf einer proximalen Seite des Konnektorrings angeordnet ist.

In einer Ausführungsform umfasst das Konnektormittel, weiter bevorzugt das Anschlussteil und/oder der Konnektorring, ein Material ausgewählt aus einer Gruppe umfassend Silikon, thermoplastische Elastomere und/oder Polyvinylchlorid. In einer Ausführungsform ist der Konnektorring materialeinheitlich mit dem Anschlussteil. In einer weiteren Ausführungsform ist der Konnektorring materialverbunden mit dem Anschlussteil. Beispielsweise ist der Konnektorring und das Anschlussteil in einem Ein- oder Zwei-Komponenten-Spritzguss gefertigt.

Der Konnektorring umfasst einen ersten Schnappring und zumindest einen zweiten Schnappring. Im Sinne der Offenbarung wird unter einem Schnappring ein erstes Teil einer Schnappverbindung verstanden. Die Schnappverbindung umfasst Funktionselemente zum lösbaren und unlösbaren, einfachen formschlüssigen Verbinden von Fügeteilen, insbesondere von Tracheostomahilfsmitteln mit dem Konnektormittel. Dabei verformt sich zumindest ein Teil der Fügeteile elastisch und verhakt anschließend bevorzugt lösbar oder unlösbar miteinander. Bevorzugt ist zumindest der Konnektorring, der Schnappring und/oder zumindest ein Teil des Tracheostomahilfsmittels elastisch verformbar. Der erste und/oder zweite Schnappring kann im Konnektorring vollständig umlaufend ausgebildet sein. In einer Ausführungsform ist der erste und/oder zweite Schnappring segmentweise ausgebildet. Insbesondere ist der erste und/oder zweite Schnappring abschnittsweise umlaufend im Konnektorring ausgebildet. Weiter bevorzugt sind erster und/oder zweiter Schnappring und Tracheostomahilfsmittel miteinander verhakbar. Bevorzugt ist die Schnappverbindung von Tracheostomahilfsmittel und ersten und/oder zweiten Schnappring lösbar. Bevorzugt sind Tracheostomahilfsmittel und Konnektormittel formschlüssig fügbar. Bevorzugt bildet der Konnektorring mit dem Tracheostomahilfsmittel eine Ringschnappverbindung. Bevorzugt ist der Konnektorring als Schnappzylinder ausgebildet. In einer Ausgestaltung ist vorgesehen, dass der Konnektorring drei oder mehr als drei Schnappringe aufweist. Vorteilhaft können damit eine Vielzahl unterschiedlicher Tracheostomahilfsmittel aufgenommen werden.

Vorteil an einer Schnappverbindung zwischen Tracheostomahilfsmittel und Konnektormittel ist, dass die Teile einfach miteinander fügbar sind. Gefügt sind die Teile jedoch insbesondere formschlüssige derart fest miteinander verbunden, dass das Tracheostomahilfsmittel beim Gebrauch nicht aus dem Konnektormittel fällt.

In einer Ausgestaltung ist vorgesehen, dass der Konnektorring zwischen dem ersten Schnappring und dem zweiten Schnappring eine erste Materialstärke aufweist und proximal des zweiten Schnapprings eine zweite Materialstärke aufweist. Hierdurch wird die Funktionalität bei gleichzeitig hinreichender Stabilität gewährleistet.

In einigen Ausgestaltungen des Tracheostomahilfsmittels umfasst dieses keinen geeigneten Hinterschnitt für eine Schnappverbindung mit dem ersten oder zweiten Schnappring. In diesem Fall wirkt der Schnappring als Klemmring. In einer Ausführungsform ist vorgesehen, dass das Tracheostomahilfsmittel mittels dem ersten oder dem zweiten Schnappring klemmend befestigbar ist, so dass eine Klemmverbindung zur Verfügung gestellt ist. Je nach Dimensionierung beziehungsweise nach geometrischer Ausführung des Tracheostomahilfsmittels wird dieses mittels einer Klemmverbindung oder einer Schnappverbindung von dem ersten oder zweiten Schnappring gehalten.

Unter Hinterschnitt des Tracheostomahilfsmittels im Sinne der Offenbarung ist zu verstehen, dass ein Durchmesser des Tracheostomahilfsmittels beziehungsweise des konischen Konnektors desselben in gegengesetzte Richtung der Fügerichtung abschnittsweise kleiner wird, so dass ein Schnappring einen Abschnitt des Tracheostomahilfsmittels beziehungsweise des konischen Konnektors hintergreifen kann.

Der erste und/oder zweite Schnappring umfassen in einer Ausführungsform zumindest eine abgeschrägte distale und/oder proximale Gleitoberfläche. Bevorzugt weist die Gleitoberfläche in einem Längsschnitt des Konnektorrings einen Winkel von etwa 30° bis etwa 50°, weiter bevorzugt etwa 38° bis etwa 48°, weiter bevorzugt etwa 45° zu einer sich in Erstreckungsrichtung des Konnektorrings erstreckenden Mittelline. Mittels der Gleitoberfläche kann das Tracheostomahilfsmittel vorteilhaft leicht in den Konnektorring eingeführt werden. In einer Ausgestaltung ist vorgesehen, dass auf der distalen Seite des ersten und/oder zweiten Schnapprings eine Gleitoberfläche vorgesehen ist, wobei die proximale Seite des ersten und/oder zweiten Schnapprings bevorzugt keine abgeschrägte distale und/oder proximale Gleitoberfläche aufweist. Bevorzugt umfasst der erste und/oder zweite Schnappring eine distale Oberfläche, die sich im Wesentlichen in einer Ebene senkrecht zu einer Mittelachse des Konnektorrings erstreckt. Vorteilhaft ist hierdurch ein Einführen des Tracheostomahilfsmittels in das Konnektormittel erleichtert, wobei das Tracheostomahilfsmittel sich nach der Einführung und dem Einschnappen hinter dem ersten und/oder zweiten Schnappring, so dass eine Verbindung der beiden Teile erfolgt, nicht so leicht entnehmen lässt beziehungsweise herausfallen kann.

Wird im Rahmen der Offenbarung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 % vorgesehen. Soweit verschiedene Wertebereiche, beispielsweise bevorzugte und weiter bevorzugte Wertebereiche, in der vorliegenden Offenbarung angegeben sind, sind die Untergrenzen und die Obergrenzen der verschiedenen Wertebereiche miteinander kombinierbar.

In einer Ausführungsform umfasst nur der zweite Schnappring eine Gleitoberfläche. Eine einer Ausführungsform umfasst nur der erste Schnappring eine Gleitoberfläche.

In einer Ausgestaltung ist vorgesehen, dass der erste Schnappring eine erste maximale Materialstärke und der zweite Schnappring eine zweite maximale Materialstärke aufweist, wobei die erste maximale Materialstärke kleiner ist als die zweite maximale Materialstärke. Bevorzugt ist eine erste minimale Materialstärke des ersten Schnapprings etwa gleich einer zweiten minimalen Materialstärke des zweiten Schnapprings. Bevorzugt umfasst der erste und/oder der zweite Schnappring minimale Materialstärke im Wesentlichen an einer radial innen gelegenen Ringinnenkante. Weiter bevorzugt weist der erste und/oder der zweite Schnappring eine maximale Materialstärke auf der Innenoberfläche des Konnektorrings zugewandten Seite auf.

Vorteilhaft ermöglicht eine Gleitoberfläche ein leichtes Fügen des Tracheostomahilfsmittels in den Konnektorring. Eine Oberfläche des Tracheostomahilfsmittels, bevorzugt ein Teil einer Schnappverbindung des Tracheostomahilfsmittels kann derart einfach über die Gleitoberfläche gleiten und dann in Fügerichtung hinter dem ersten und/oder zweiten Schnappring einrasten. Auch wenn das Tracheostomahilfsmittel keine geeignete Geometrie für das Einrasten des ersten und/oder zweiten Schnapprings aufweist, kann die Gleitoberfläche ein einfaches Fügen des Tracheostomahilfsmittels in den Konnektorring ermöglichen, wobei das Tracheostomahilfsmittel mit geringem Kraftaufwand in eine Klemmposition gebracht werden kann. Ein weiterer Vorteil der insbesondere trichterförmigen Gleitoberfläche ist, dass das Tracheostomahilfsmittel beim Einführen in den Konnektorring zentriert wird.

Bevorzugt ist der Schnappring als zumindest teilweise umlaufender Ring oder eine Anzahl von Ringsegmenten ausgebildet. Offenbarungsgemäß umfasst der erste und/oder zweite Schnappring eine wulstförmige Materialanhäufung. Weiter bevorzugt ist der erste und/oder zweite Schnappring trichterförmig ausgestaltet. Im Sinne der Offenbarung ist unter "trichterförmig" eine Ausgestaltung insbesondere einer distalen Oberfläche des ersten und/oder zweiten Schnapprings zu verstehen, die zur Mittelachse und nach proximal geneigt ist. Bevorzugt ist der erste und/oder der zweite Schnappring als sich nach radial innen erstreckender Vorsprung ausgestaltet. Weiter bevorzugt umfasst der erste und/oder zweite Schnappring eine Ringscheibe. Im Sinne der Offenbarung ist eine distale Oberfläche und eine proximale Oberfläche des ersten und/oder zweiten Schnapprings im Wesentlichen jeweils auf einer Ebene senkrecht zur Mittelachse des Konnektorrings angeordnet. Bevorzugt sind eine distale Oberfläche und eine proximale Oberfläche des ersten und/oder zweiten Schnapprings im Wesentlichen parallel zueinander angeordnet. Weiter bevorzugt ist eine distale oder eine proximale Oberfläche des ersten und/oder zweiten Schnapprings im Wesentlichen jeweils auf einer Ebene senkrecht zur Mittelachse des Konnektorrings angeordnet.

Der Begriff "im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

In einer Ausführungsform ist eine proximale Oberfläche des ersten Schnapprings auf einer Ebene senkrecht zur Mittelachse des Konnektorrings angeordnet und eine proximale Oberfläche des zweiten Schnapprings trichterförmig ausgestaltet. In einer weiteren Ausgestaltung sind die proximalen Oberflächen des ersten und des zweiten Schnapprings trichterförmig ausgestaltet. In einer Ausführungsform ist vorgesehen, dass die proximale Seite des ersten und/oder zweiten Schnapprings im Wesentlichen auf einer Ebene senkrecht zur Mittelachse des Konnektorrings angeordnet ist. In einer Ausführungsform ist vorgesehen, dass ein Betrag eines Winkels der proximalen Seite des ersten und/oder zweiten Schnapprings zu der Ebene senkrecht zur Mittelachse kleiner ist als ein Betrag eines Winkel der entsprechenden distalen Seite zu der Ebene senkrecht zur Mittelachse.

Vorteilhaft kann insbesondere ein konischer Konnektor eines Tracheostomahilfsmittels auf der trichterförmigen beziehungsweise abgeschrägten distalen Seite des ersten und/oder zweiten Schnapprings abgleiten und dabei über die distale Seite eine Kraft in den Konnektorring einleiten, die diesen verformt, bevorzugt um das Tracheostomahilfsmittel zu klemmen oder in einen Hinterschnitt zu schnappen. Wenn das Tracheostomahilfsmittl beziehungsweise der konische Konnektor einen Hinterschnitt aufweist, kann nachdem der Hinterschnitt den ersten oder zweiten Schnappring überstreicht, der erste oder zweite Schnappring zurück schnappen und mit dem konischen Konnektor eine formschlüssige Schnappverbindung bilden. Beim Herausziehen des Tracheostomahilfsmittels wird eine Kraft von dem konischen Konnektor in die proximale Seite des ersten oder zweiten Schnapprings eingeleitet. Wenn die proximale Seite des ersten und/oder zweiten Schnapprings im Wesentlichen auf einer Ebene senkrecht zur Mittelachse des Konnektorrings angeordnet ist oder ein Betrag eines Winkels dieser proximalen Seite zu der Ebene senkrecht zur Mittelachse kleiner ist als ein Betrag eines Winkel der entsprechenden distalen Seite zu der Ebene senkrecht zur Mittelachse, ist die Zugkraft zum Entfernen des Tracheostomahilfsmittels aus dem Konnektorring wesentlich größer als die Druckkraft zum Fügen des Tracheostomahilfsmittels in den Konnektorring.

Der erste und/oder der zweite Schnappring weisen einen Innendurchmesser von etwa 11 mm bis etwa 25 mm, bevorzugt etwa 21 mm bis etwa 24 mm, weiter bevorzugt etwa 11,5 mm, weiter bevorzugt etwa 15 mm, weiter bevorzugt etwa 16 mm, weiter bevorzugt etwa 22 mm, weiter bevorzugt etwa 23 mm auf.

In einer Ausführungsform umfasst der erste Schnappring einen ersten Innendurchmesser. In einer Ausgestaltung ist vorgesehen, dass der erste Schnappring einen ersten Innendurchmesser von etwa 16 mm oder etwa 23 mm aufweist.

In einer Ausführungsform umfasst der zweite Schnappring einen zweiten Innendurchmesser. Bevorzugt ist der zweite Innendurchmesser kleiner als der erste Innendurchmesser. In einer Ausgestaltung ist vorgesehen, dass der zweite Schnappring einen zweiten Innendurchmesser von etwa 15 mm oder etwa 22 mm aufweist.

In einer Ausführungsform ist der erste Innendurchmesser des ersten Schnapprings etwa 16 mm und der zweite Innendurchmesser des zweiten Schnapprings etwa 15 mm. In einer Ausführungsform ist der erste Innendurchmesser des ersten Schnapprings etwa 23 mm und der zweite Innendurchmesser des zweiten Schnapprings etwa 22 mm. Bevorzugt beträgt der Unterschied zwischen ersten und zweiten Innendurchmesser etwa 1 mm bis etwa 2 mm.

Offenbarungsgemäß ist vorgesehen, dass der erste Schnappring einen ersten Innendurchmesser und der zweite Schnappring einen zweiten Innendurchmesser aufweist, wobei der erste Innendurchmesser größer ist als der zweite Innendurchmesser.

Offenbarungsgemäß ist vorgesehen, dass der erste Schnappring distal von dem zweiten Schnappring angeordnet ist. Vorteilhaft kann ein Tracheostomahilfsmittel mit einem kleineren Außendurchmesser als derjenige des ersten Schnapprings an dem ersten Schnappring vorbeigeführt werden und ist mit dem zweiten Schnappring insbesondere formschlüssig oder kraftschlüssig verbindbar.

Bevorzugt ist der zweite Schnappring proximal vom ersten Schnappring angeordnet. In einer Ausgestaltung ist vorgesehen, dass der erste Schnappring an einem distalen Ende des Konnektorrings angeordnet ist. Dies ermöglicht vorteilhaft, dass das Tracheostomahilfsmittel, das mit dem ersten Schnappring klemmend oder schnappend verbunden ist, auf dem zweiten Schnappring aufsitzen kann.

In einer Ausführungsform umfasst das Konnektormittel einen Respirationsschutz. Weiter bevorzugt umfasst der Respirationsschutz mindestens einen Steg, ein Gitter und/oder einen, bevorzugt umlaufenden, Absatz. In einer Ausführungsform umfasst der Respirationsschutz eine bevorzugt zumindest teilweise umlaufende Materialanhäufung bevorzugt auf der Innenseite des Konnektorrings. Bevorzugt ist der Respirationsschutz als Ring oder Ringscheibe ausgestaltet. Vorteilhaft verhindert der Respirationsschutz ein zu weites Durchstecken des Tracheostomahilfsmittels. Bevorzugt umfasst der Respirationsschutz einen kleineren Innendurchmesser als der der erste und der zweite Schnappring. Bevorzugt ist der Respirationsschutz an einem proximalen Ende des Konnektormittels, weiter bevorzugt am proximalen Ende des Konnektorrings angeordnet. Bevorzugt ist der Respirationsschutz im Wesentlichen eben mit einer proximalen Seite des Anschlussteils ausgestaltet, insbesondere bei Ausgestaltung des Anschlussteils als Basisplatte. Bevorzugt ist der Respirationsschutz mit der Basisplatte materialverbunden oder materialeinheitlich ausgestaltet. Bevorzugt ist der Respirationsschutz mit dem Konnektorring materialverbunden oder materialeinheitlich ausgestaltet.

In einer beispielhaften Ausführungsform des Konnektormittels umfasst dieses ein Anschlussteil. Das Anschlussteil ist beispielhaft als Basisplatte eines Pflasters ausgebildet. Das Anschlussteil ist beispielhaft auf einem Klebemittel angeordnet, das als Klebefolie ausgestaltet ist. Am proximalen Ende des Konnektorrings erstreckt sich nach radial außen das Anschlussteil. Der Konnektorring ist hohlzylindrisch ausgestaltet und umfasst eine Innenoberfläche. Auf der Innenoberfläche ist ein erster Schnappring und ein zweiter Schnappring ausgebildet. Der erste Schnappring ist beispielhaft einem distalen Ende des Konnektorrings zugeordnet. Der erste Schnappring ist beispielhaft ringscheibenförmig ausgestaltet und bündig mit einem distalen Ende des Konnektorrings angeordnet. Der erste Schnappring weist beispielhaft einen ersten Innendurchmesser von etwa 23 mm auf. Proximal vom ersten Schnappring und in Richtung der Mittelachse des Konnektorrings etwa auf halber Höhe ist der zweite Schnappring angeordnet. Der zweite Schnappring umfasst distal eine Gleitoberfläche, die in einem Winkel von etwa 45° zu einer Mittelachse des Konnektorrings abgeschrägt ist. Die proximale Seite des zweiten Schnappring erstreckt sich beispielhaft auf einer Ebene senkrecht zur Mittelachse des Konnektorrings. Der zweite Schnappring weist beispielhaft einen zweiten Innendurchmesser gemäß DIN EN ISO 5356-1:2015-06 von etwa 22 mm auf. Am proximalen Ende des Konnektorrings ist ein Respirationsschutz angeordnet. Der Respirationsschutz ist auf der proximalen Seite bündig mit dem Anschlussteil ausgestaltet. Der Respirationsschutz ist ringscheibenförmig auf der Innenoberfläche des Konnektorrings angeordnet. Der Konnektorring, der erste Schnappring, der zweite Schnappring und das Anschlussteil sind materialeinheitlich ausgebildet.

Ein beispielhaftes erstes Tracheostomahilfsmittel nach DIN EN ISO 5356-1:2015-06 mit einem Durchmesser von etwa 22 mm umfasst einen konischen Konnektor der einen Hinterschnitt ausbildet, der mit dem zweiten Schnappring zusammen wirken kann. Bei einem beispielhaften Fügen des ersten Tracheostomahilfsmittels in den Konnektorring des Konnektormittels wird dieses an dem ersten Schnappring vorbeigeführt, ohne mit diesem zu interagieren, da der erste Schnappring einen ersten Innendurchmesser aufweist, der größer ist als ein maximaler Außendurchmesser des konischen Konnektors. Beim weiteren Fügen wird er konische Konnektor beispielhaft an dem zweiten Schnappring vorbeigeführt, wobei der zweite Schnappring beziehungsweise der Konnektorring elastisch verformt wird. Nachdem der Hinterschnitt des konischen Konnektors den zweiten Schnappring überstrichen hat, schnappt dieser beispielhaft wieder im Wesentlichen in seine ursprüngliche Form und hält das erste Tracheostomahilfsmittel formschlüssig im Konnektormittel. Nach proximal wird die Fügung des ersten Tracheostomahilfsmittels beispielhaft durch den Respirationsschutz, der auf der Höhe des Anschlussteils angeordnet ist, begrenzt.

Ein beispielhaftes zweites Tracheostomahilfsmittel weist einen konischen Konnektor mit einem Außendurchmesser auf, der größer als der Außendurchmesser des ersten Tracheostomahilfsmittels ist. Beim Fügen des zweiten Tracheostomahilfsmittels in den Konnektorring wird der konische Konnektor an dem ersten Schnappring derart vorbeigeführt, dass der erste Schnappring beziehungsweise der Konnektorring elastisch verformt wird. Nachdem ein Hinterschnitt des konischen Konnektors den ersten Schnappring überstrichen hat, schnappt dieser wieder im Wesentlichen in seine ursprüngliche Form und hält das zweite Tracheostomahilfsmittel beispielhaft formschlüssig im Konnektormittel. Das zweite Tracheostomahilfsmittel kann auf dem zweiten Schnappring aufsitzen, womit eine übermäßige Bewegung des zweiten Tracheostomahilfsmittels in dem Konnektormittel begrenzt wird.

Ein beispielhaftes drittes Tracheostomahilfsmittel nach DIN EN ISO 5356-1:2015-06 mit einem Durchmesser von etwa 22 mm umfasst einen konischen Konnektor, der keinen Hinterschnitt ausbildet. Bei einem beispielhaften Fügen des dritten Tracheostomahilfsmittels in den Konnektorring des Konnektormittels wird dieses an dem ersten Schnappring vorbeigeführt, ohne mit diesem zu interagieren, da der erste Schnappring einen ersten Innendurchmesser aufweist, der größer ist als ein maximaler Außendurchmesser des konischen Konnektors. Beim weiteren Fügen wird der konische Konnektor beispielhaft an dem zweiten Schnappring vorbeigeführt, wobei der zweite Schnappring beziehungsweise der Konnektorring elastisch verformt wird. Das Fügen wird beendet, wenn der zweite Schnappring mit einer Außenoberfläche des konischen Konnektors einen Reibwiderstand bildet, der für den Benutzer als für eine Klemmung ausreichend empfunden wird. Alternativ wird das Fügen beendet, wenn die Kraft zum Fügen des dritten Tracheostomahilfsmittels in den Konnektorring etwa 50 N übersteigt. Nach proximal wird die Fügung des ersten Tracheostomahilfsmittels beispielhaft durch den Respirationsschutz, der auf der Höhe des Anschlussteils angeordnet ist, begrenzt.

Weiterhin wird ein Pflaster mit einem oben beschriebenen Konnektormittel vorgeschlagen, wobei das Anschlussteil auf einer distalen Seite des Pflasters angeordnet ist. Bevorzugt umfasst das Pflaster ein Klebemittel, das auf der proximalen Seite des Anschlussteils aufgebracht ist. Beispielsweise ist das Klebemittel als eine Klebefolie ausgestaltet. In einer Ausgestaltung überragt das Klebemittel beziehungsweise die Klebefolie das Anschlussteil auf zumindest einer Seite, bevorzugt nach allen Seiten auf einer Ebene, in der das Klebemittel angeordnet ist.

Weiterhin wird eine Trachealkanüle mit einem oben beschriebenen Konnektormittel vorgeschlagen, wobei ein Konnektorring an einem distalen Ende eines Kanülenrohrs angeordnet ist. Die Trachealkanüle kann als Innenkanüle oder Außenkanüle ausgestaltet sein. Insbesondere bei einer Ausgestaltung als Innenkanüle kann das Kanülenrohr gefenstert, gesiebt oder ungefenstert ausgestaltet sein.

Weiterhin wird eine Verwendung eines oben beschriebenen Konnektormittel zur Verbindung mit Tracheostomahilfsmitteln mit unterschiedlichen Außendurchmessern vorgeschlagen.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
- Fig. 1: eine perspektivische Ansicht eines Pflasters mit einem Konnektormittel;
- Fig. 2: eine Ansicht eines Längsschnittes eines Konnektormittels;
- Fig. 3: eine Ansicht eines Längsschnittes des Konnektormittels aus Fig. 2 mit einem ersten Tracheostomahilfsmittel;
- Fig. 4: eine Ansicht eines Längsschnittes des Konnektormittels aus Fig. 2 mit einem zweiten Tracheostomahilfsmittel; und
- Fig. 5: eine Trachealkanüle mit einem Konnektormittel.

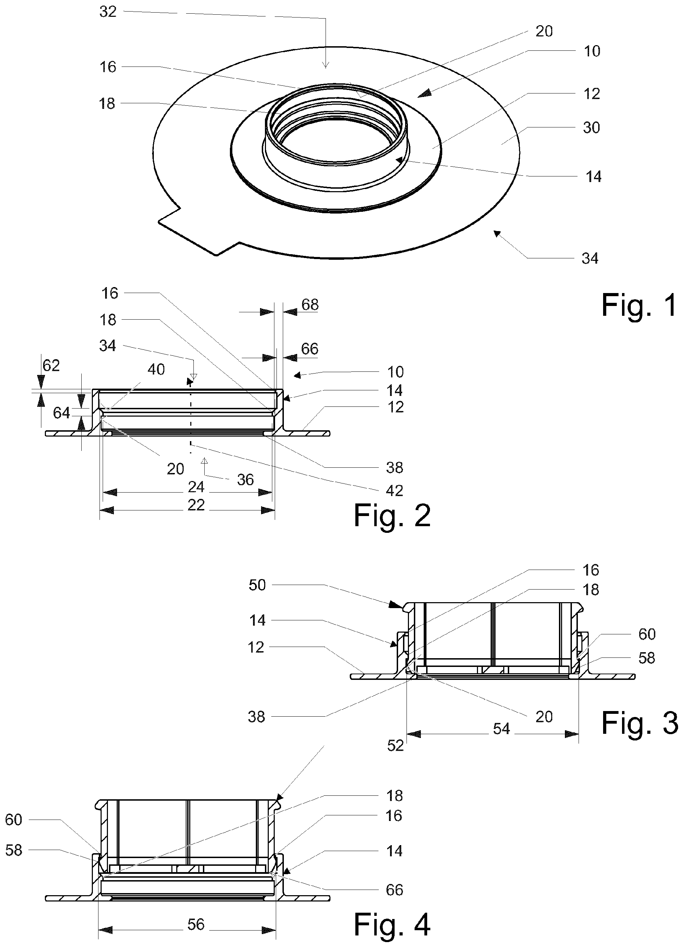

Fig. 1 zeigt eine perspektivische Ansicht eines Pflasters 34 mit einem Konnektormittel 10. Ein Anschlussteil 12 ist als Basisplatte eines Pflasters 34 ausgebildet. Das Anschlussteil 12 ist auf einem Klebemittel 30 angeordnet, das als Klebefolie mit einer abziehbaren Schutzfolie ausgestaltet ist. Von dem Anschlussteil 12 erstreckt sich nach distal ein Konnektorring 14. Der Konnektorring 14 ist hohlzylindrisch ausgestaltet und umfasst eine Innenoberfläche 20. Auf der Innenoberfläche 20 ist eine erster Schnappring 16 und ein zweiter Schnappring 18 ausgebildet. Der erste Schnappring 16 ist einem distalen Ende 32 des Konnektorrings 14 zugeordnet. Proximal vom ersten Schnappring 16 und in Richtung der Längsachse des Konnektorrings etwa auf halber Höhe ist der zweite Schnappring 18 angeordnet.

Fig. 2 zeigt eine Ansicht eines Längsschnittes eines Konnektormittels 10 ohne das in Fig. 1 gezeigte Pflaster 34. Der erste Schnappring 16 ist bündig mit einem distalen Ende 32 des Konnektorrings 14 ausgebildet. Der erste Schnappring 16 weist einen ersten Innendurchmesser 22 von etwa 23 mm auf. Der erste Schnappring 16 umfasst eine erste maximale Materialstärke 62. Weiterhin ist der zweite Schnappring 18 etwa mittig auf der Innenoberfläche 20 des Konnektorrings 14 ausgebildet. Der zweite Schnappring 18 umfasst distal eine Gleitoberfläche 40, die in einem Winkel von etwa 45° zu einer Mittelachse 42 abgeschrägt ist, wobei der Winkel der Übersichtlichkeit halber nicht eingezeichnet ist. Der zweite Schnappring 18 umfasst eine zweite maximale Materialstärke 64, wobei die erste maximale Materialstärke 62 kleiner ist als die zweite maximale Materialstärke 64. Der zweite Schnappring weist einen zweiten Innendurchmesser 24 gemäß DIN EN ISO 5356-1:2015-06 von etwa 22 mm auf. Am proximalen Ende 36 des Konnektorrings 14 ist ein Respirationsschutz 38 angeordnet. Der Respirationsschutz 38 ist auf der proximalen Seite 36 bündig mit dem Anschlussteil 12 ausgebildet. Der Respirationsschutz 38 ist als Ringscheibe auf der Innenoberfläche 20 des Konnektorrings 14 angeordnet. Der Konnektorring 14, der erste Schnappring 16, der zweite Schnappring 18 und das Anschlussteil 12 sind materialeinheitlich ausgebildet.

Fig. 3 zeigt eine Ansicht eines Längsschnittes des Konnektormittels 10 aus Fig. 2 mit einem ersten Tracheostomahilfsmittel 50 nach DIN EN ISO 5356-1:2015-06 mit einem Durchmesser von etwa 22 mm. Das erste Tracheostomahilfsmittel 50 umfasst einen konischen Konnektor 58, der einen Hinterschnitt 60 ausbildet, der mit dem zweiten Schnappring 18 zusammen wirkt. Bei einem Fügen des ersten Tracheostomahilfsmittels 50 in den Konnektorring 14 wird dieses an dem ersten Schnappring 16 vorbeigeführt, ohne mit diesem zu interagieren, da der erste Schnappring 16 einen in Fig. 2 gezeigten ersten Innendurchmesser 22 aufweist, der größer ist als ein maximaler Außendurchmesser 54 des konischen Konnektors 58. Beim weiteren Fügen wird der konische Konnektor 58 an dem zweiten Schnappring 18 derart vorbeigeführt, dass der zweite Schnappring 18 beziehungsweise der Konnektorring 14 elastisch verformt wird. Nachdem der Hinterschnitt 60 des konischen Konnektors 58 den zweiten Schnappring 18 überstrichen hat, schnappt dieser wieder im Wesentlichen in seine ursprüngliche Form und hält das erste Tracheostomahilfsmittel 50 formschlüssig im Konnektormittel 10. Nach distal wird die Fügung des ersten Tracheostomahilfsmittels 50 durch den Respirationsschutz 38, der auf der Höhe des Anschlussteils 12 angeordnet ist, begrenzt.

Fig. 4 zeigt eine Ansicht eines Längsschnittes des Konnektormittels 10 aus Fig. 2 mit einem zweiten Tracheostomahilfsmittel 52. Das zweite Tracheostomahilfsmittel 52 weist einen konischen Konnektor 58 mit einem Außendurchmesser 56 auf. Der Außendurchmesser 56 des zweiten Tracheostomahilfsmittels 52 ist größer als der Außendurchmesser 54 des ersten Tracheostomahilfsmittels 50. Beim Fügen des zweiten Tracheostomahilfsmittels 52 in den Konnektorring 14 wird der konische Konnektor 58 an dem ersten Schnappring 16 derart vorbeigeführt, dass der erste Schnappring 16 beziehungsweise der Konnektorring 14 elastisch verformt wird. Nachdem der Hinterschnitt 60 des konische Konnektors 58 den ersten Schnappring 16 überstrichen hat, schnappt dieser wieder im Wesentlichen in seine ursprüngliche Form und hält das zweite Tracheostomahilfsmittel 52 formschlüssig im Konnektormittel 10. Das zweite Tracheostomahilfsmittel 52 kann mit seinem distalen Ende 66 auf dem zweiten Schnappring 18 aufsitzen, womit eine übermäßige Bewegung des zweiten Tracheostomahilfsmittels 52 in dem Konnektormittel 10 begrenzt wird.

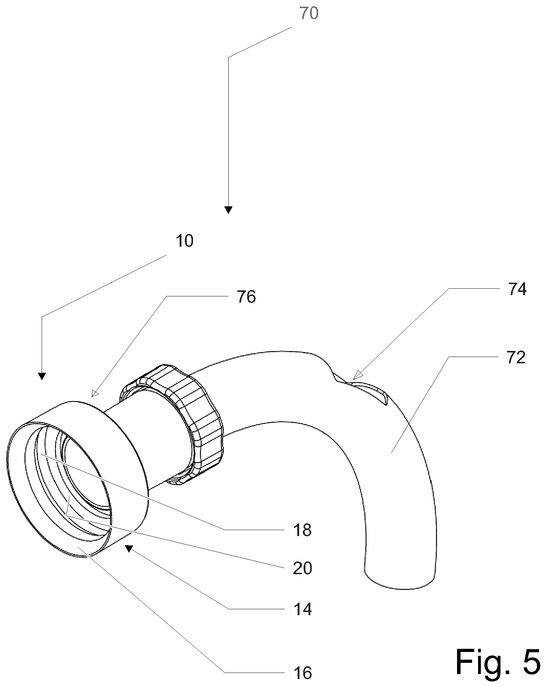

Fig. 5 zeigt eine Trachealkanüle 70 mit einem Konnektormittel 10. Die Trachealkanüle 70 ist als Innenkanüle ausgebildet und weist eine Fensterung 74 auf. Wie in der allgemeinen Beschreibung ausgeführt, kann die Trachealkanüle auch ungefenstert, gesiebt und/oder als Außenkanüle ausgestaltet sein. Die Trachealkanüle 70 weist am distalen Ende eines Kanülenrohrs 72 das Konnektormittel 10 mit einem Konnektorring 14 auf. Der Konnektorring 14 umfasst einen ersten Schnappring 16 und zumindest einen zweiten Schnappring 18, wobei der erste Schnappring 16 und der zweite Schnappring 18 auf einer Innenoberfläche 20 des Konnektorrings 14 ausgebildet sind. Der erste Schnappring 16 weist einen ersten Innendurchmesser von etwa 23 mm auf, und der zweite Schnappring weist einen zweiten Innendurchmesser gemäß DIN EN ISO 5356-1:2015-06 von etwa 22 mm auf.

Vorteilhaft können mittels des vorgeschlagenen Konnektormittels 10 Tracheostomahilfsmittel 50, 52 mit unterschiedlichen Nennmaßen aufgenommen werden. Insbesondre kann das Konnektormittel vorteilhaft mit Tracheostomahilfsmitteln 50 nach DIN EN ISO 5356-1:2015-06 und mit ungenormten Tracheostomahilfsmitteln 52 verwendet werden.

## Patentansprüche

1. Konnektormittel (10) zur Verbindung mit einem Tracheostomahilfsmittel (50, 52), wobei das Konnektormittel (10) einen Konnektorring (14) umfasst, wobei der Konnektorring (14) einen ersten Schnappring (16) und zumindest einen zweiten Schnappring (18) umfasst, wobei der erste Schnappring (16) und der zweite Schnappring (18) auf einer Innenoberfläche (20) des Konnektorrings (14) ausgebildet sind und wobei der erste Schnappring (16) einen ersten Innendurchmesser (22) und der zweite Schnappring (18) einen zweiten Innendurchmesser (24) aufweist, wobei der erste Innendurchmesser (22) größer ist als der zweite Innendurchmesser (24), **dadurch gekennzeichnet, dass** der erste Schnappring (16) und der zweite Schnappring (18) eine wulstförmige Materialanhäufung umfassen und elastisch verformbar sind und wobei der erste Schnappring (16) distal, das heißt bei üblichen Gebrauch des Konnektormittels (10) in einer Richtung vom Körper einer Person weg gerichtet, von dem zweiten Schnappring (18) angeordnet ist.

2. Konnektormittel (10) gemäß Anspruch 1, **dadurch gekennzeichnet, dass** dieses ein Anschlussteil (12) umfasst, das als eine Basisplatte ausgebildet ist.

3. Konnektormittel (10) gemäß einem oder mehreren der vorhergehenden Ansprüche, **dadurch gekennzeichnet, dass** proximal des Konnektorrings (14) ein Kanülenrohr (70) angeordnet ist.

4. Konnektormittel (10) gemäß einem oder mehreren der vorhergehenden Ansprüche, **dadurch gekennzeichnet, dass** der erste Schnappring (16) an einem distalen Ende (28) des Konnektorrings (14) angeordnet ist.

5. Konnektormittel (10) gemäß einem oder mehreren der vorhergehenden Ansprüche, **dadurch gekennzeichnet, dass** der Konnektorring (14) zwischen dem ersten Schnappring (16) und dem zweiten Schnappring (18) eine erste Materialstärke (66) aufweist und proximal des zweiten Schnapprings (18) eine zweite Materialstärke (68) aufweist.

6. Konnektormittel (10) gemäß einem oder mehreren der vorhergehenden Ansprüche, **dadurch gekennzeichnet, dass** der erste Schnappring (16) einen ersten Innendurchmesser von etwa 16 mm oder etwa 23 mm aufweist.

7. Konnektormittel (10) gemäß einem oder mehreren der vorhergehenden Ansprüchen, **dadurch gekennzeichnet, dass** der zweite Schnappring (18) einen zweiten Innendurchmesser von etwa 15 mm oder etwa 22 mm aufweist.

8. Pflaster (34) mit einem Konnektormittel (10) gemäß einem oder mehreren der vorhergehenden Ansprüche, wobei ein Anschlussteil (12) auf einer distalen Seite des Pflasters (34) angeordnet ist.

9. Trachealkanüle (70) mit einem Konnektormittel (10) gemäß einem oder mehreren der Ansprüche 1 bis 7, wobei ein Konnektorring (14) an einem distalen Ende (76) eines Kanülenrohrs (72) angeordnet ist.

10. Verwendung eines Konnektormittels (10) gemäß einem oder mehreren der Ansprüche 1 bis 7 zur Verbindung mit Tracheostomahilfsmitteln (50, 52) mit unterschiedlichen Außendurchmessern (54, 56).

## Claims

1. A connector means (10) for connection to a tracheostoma aid (50, 52), wherein the connector means (10) comprises a connector ring (14), wherein the connector ring (14) comprises a first snap ring (16) and at least one second snap ring (18), wherein the first snap ring (16) and the second snap ring (18) are formed on an inner surface (20) of the connector ring (14) and wherein the first snap ring (16) has a first inner diameter (22) and the second snap ring (18) has a second inner diameter (24), wherein the first inner diameter (22) is larger than the second inner diameter (24), **characterized in that** the first snap ring (16) and the second snap ring (18) comprise a bead-shaped material accumulation and are elastically deformable and wherein the first snap ring (16) is arranged distally, that is, directed in a direction away from the body of an individual during normal use of the connector means (10), from the second snap ring (18).

2. The connector means (10) according to claim 1, **characterized in that** the latter comprises a connecting piece (12), which is formed as a base plate.

3. The connector means (10) according to one or several of the preceding claims, **characterized in that** a cannula tube (70) is arranged proximally from the connector ring (14).

4. The connector means (10) according to one or several of the preceding claims, **characterized in that** the first snap ring (16) is arranged on a distal end (28) of the connector ring (14).

5. The connector means (10) according to one or several of the preceding claims, **characterized in that** the connector ring (14) has a first material thickness (66) between the first snap ring (16) and the second snap ring (18) and has a second material thickness (68) proximally from the second snap ring (18).

6. The connector means (10) according to one or several of the preceding claims, **characterized in that** the first snap ring (16) has a first inner diameter of approximately 16 mm or approximately 23 mm.

7. The connector means (10) according to one or several of the preceding claims, **characterized in that** the second snap ring (18) has a second inner diameter of approximately 15 mm or approximately 22 mm.

8. A tape (34) with a connector means (10) according to one or several of the preceding claims, wherein a connecting piece (12) is arranged on a distal side of the tape (34).

9. A tracheal cannula (70) with a connector means (10) according to one or several of claims 1 to 7, wherein a connector ring (14) is arranged on a distal end (76) of a cannula tube (72).

10. Use of a connector means (10) according to one or several of claims 1 to 7 for connection to tracheostomy aids (50, 52) with different outer diameters (54, 56).

## Revendications

1. Dispositif de connexion (10) destiné à être raccordé à un dispositif de trachéotomie (50, 52), le dispositif de connexion (10) comprenant une bague de connexion (14), laquelle bague de connexion (14) comprenant un premier anneau à encliquetage (16) et au moins un deuxième anneau à encliquetage (18), le premier anneau à encliquetage (16) et le deuxième anneau à encliquetage (18) étant formés sur une surface intérieure (20) de la bague de connexion (14), le premier anneau à encliquetage (16) présentant un premier diamètre intérieur (22) et le deuxième anneau à encliquetage (18) un deuxième diamètre intérieur (24), le premier diamètre intérieur (22) étant supérieur au deuxième diamètre intérieur (24), **caractérisé en ce que** le premier anneau à encliquetage (16) et le deuxième anneau à encliquetage (18) comprennent un renflement de matière et sont déformables élastiquement, et dans lequel le premier anneau à encliquetage (16) est situé distal, c'est-à-dire orienté, lors d'une utilisation normale du dispositif de connexion (10), dans une direction s'éloignant du corps d'une personne, par rapport au deuxième anneau à encliquetage (18).

2. Dispositif de connexion (10) selon la revendication 1, **caractérisé en ce qu'**il comprend une pièce de raccordement (12) qui est réalisée sous la forme d'une plaque de base.

3. Dispositif de connexion (10) selon une ou plusieurs des revendications précédentes, **caractérisé en ce qu'**un tube de canule (70) est disposé proximal de la bague de connexion (14).

4. Dispositif de connexion (10) selon une ou plusieurs des revendications précédentes, **caractérisé en ce que** le premier anneau à encliquetage (16) est disposé à une extrémité distale (28) de la bague de connexion (14).

5. Dispositif de connexion (10) selon une ou plusieurs des revendications précédentes, **caractérisés en ce que** la bague de connexion (14) présente une première épaisseur de matériau (66) entre le premier anneau à encliquetage (16) et le deuxième anneau à encliquetage (18), et une deuxième épaisseur de matériau (68) proximale du deuxième anneau à encliquetage (18).

6. Dispositif de connexion (10) selon une ou plusieurs des revendications précédentes, **caractérisé en ce que** le premier anneau à encliquetage (16) présente un premier diamètre intérieur d'environ 16 mm ou d'environ 23 mm.

7. Dispositif de connexion (10) selon une ou plusieurs des revendications précédentes, **caractérisé en ce que** le deuxième anneau à encliquetage (18) présente un deuxième diamètre intérieur d'environ 15 mm ou d'environ 22 mm.

8. Pansement (34) comprenant un dispositif de connexion (10) selon une ou plusieurs des revendications précédentes, dans lequel une pièce de raccordement (12) est disposée sur un côté distal du pansement (34).

9. Canule trachéale (70) comprenant un dispositif de connexion (10) selon une ou plusieurs des revendications 1 à 7, dans laquelle une bague de connexion (14) est disposée à une extrémité distale (76) d'un tube de canule (72).

10. Utilisation d'un dispositif de connexion (10) selon une ou plusieurs des revendications 1 à 7 pour la connexion à des dispositifs de trachéotomie (50, 52) présentant différents diamètres extérieurs (54, 56).
